# EUROPÄISCHE PATENTSCHRIFT

(11) **EP 1 284 757 B1**
(45) Veröffentlichungstag und Bekanntmachung des Hinweises auf die Patenterteilung: **25.02.2004**
(21) Anmeldenummer: 01936368.8
(22) Anmeldetag: 17.05.2001
(51) Int. Cl.: A61L 31/16, A61L 31/08, A61K 51/12, C23C 14/16

(54) **SYSTEM MIT EINEM TRÄGERSUBSTRAT UND EINER Ti/P-BZW. AI/P-BESCHICHTUNG**
SYSTEM COMPRISING A CARRIER SUBSTRATE AND A TI/P OR. AI/P COATING
SYSTEME COMPORTANT UN SUBSTRAT SERVANT DE SUPPORT ET UN REVETEMENT DE TI/P OU AI/P

(30) Priorität: 29.05.2000 DE 10026485
(43) Veröffentlichungstag der Anmeldung: 26.02.2003
(73) Patentinhaber: Grabowy, Udo, 53881 Emskirchen-Lamersheim (DE); Busch, Heinz Werner, 53639 Königswinter-Thomasberg (DE)
(72) Erfinder: Grabowy, Udo, 53881 Emskirchen-Lamersheim (DE); Busch, Heinz Werner, 53639 Königswinter-Thomasberg (DE)
(74) Vertreter: Dr. Weitzel & Partner
(86) Internationale Anmeldenummer: PCT/EP2001/005610
(87) Internationale Veröffentlichungsnummer: WO 2001/091823

(56) Entgegenhaltungen:
- US-A- 5 871 437
- US-A- 5 919 126
- US-A- 6 010 445

## Beschreibung

Die Erfindung betrifft ein System mit einem Substrat als Träger und einer auf das Substrat aufgebrachten Beschichtung sowie ein Verfahren zum Aufbringen einer derartigen Beschichtung in einer Sputteranlage, insbesondere in einer PVD- bzw. PECVD-Sputteranlage und ein Implantat, insbesondere Gefäßimplantat, umfassend ein derartiges System bzw. eine Beschichtung, hergestellt durch Aufsputtern in einer solchen Sputteranlage.

Systeme mit einem Substrat als Träger und auf das Substrat aufgebrachte Beschichtungen sind in vielen Bereichen der Technik, beispielsweise der Telekommunikationstechnik bekannt.

Diesbezüglich wird beispielsweise auf die nachfolgenden Druckschrift "Vakuumbeschichtung 1, Plasmaphysik - Plasmadiagnostik - Analytik", herausgegeben von Dr.Ing. Hartmut Frey, VDI Verlag, Düsseldorf 1995 verwiesen. Zum Aufbringen der Beschichtung auf einen Substratträger sind eine Vielzahl von Verfahren bekanntgeworden. Ein Verfahren zum Aufbringen von Beschichtungen ist das Aufsputtem. Gemäß dem Lexikon "Elektronik und Mikroelektronik", herausgegeben von Dieter Sautter und Hans Weinerth, VDI-Verlag 1990, S.804-807, versteht man unter Sputtern oder Kathodenzerstäuben einen physikalischen Prozess, mit dem jedes als Festkörper vorliegende Material auf einem beliebigen Substrat als Schicht mit guter Haftung abgeschieden werden kann. In der Silizium-Halbleitertechnologie werden mit Sputterprozessen vor allem Metallisierungsschichten für Kontakte und Leiterbahnen sowie Passivierungschichten zum Schutz von fertigen integrierten Schaltungen hergestellt.

Durch den Sputterprozess selbst werden die zu beschichtenden Träger nur auf relativ niedrige Temperaturen aufgeheizt.

Das Prinzip des Aufsputterns funktioniert derart, dass in einer Vakuumkammer bei niedrigem Druck ein Edelgasplasma, bevorzugt Argonplasma, erzeugt wird, die positiv geladenen Edelgasionen aus dem Plasma zu dem auf negativem Potential liegenden Target, welches aus dem abzuscheidenden Material besteht, aufgrund des elektrischen Feldes beschleunigt werden, und durch das Auftreffen der Edelgasionen aus dem Target das Material herausgeschlagen wird, das sich auf der gegenüberliegenden Oberfläche auf dem zu beschichtenden Substrat abscheidet.

Es gibt eine Vielzahl verschiedener Sputteranlagen, z.B. Gleichspannungs-Planar-Diodensputteranlagen, Hochfrequenzsputteranlagen sowie BIAS-Sputteranlagen. Betreffend die Ausgestaltung unterschiedlicher Sputteranlagen wird wiederum auf "Lexikon Elektronik und Mikroelektronik aaO" verwiesen. Um eine hohe Plasmadichte direkt an der Targetoberfläche zu erreichen, können sogenannte Magnetrontargets verwendet werden, die sich dadurch auszeichnen, dass durch die geschickte Anbringung eines Magnetfeldes nahe der Targetoberfläche eine hohe Plasmadichte direkt an der Targetoberfläche innerhalb des Bereiches, in dem das Magnetfeld stark genug ist, erreicht wird. Durch Verwendung eines Magnetrontargets kann somit die Sputterrate bei gleichem Argondruck gegenüber der Verwendung eines normalen Targets ganz entscheidend erhöht werden. Durch Variation des Gasdrucks können bei gleichbleibender Sputterrate die Schichteigenschaften verändert werden.

Betreff die verschiedenen Sputtertechniken wird auf "Lexikon Elektronik und Mikroelektronik aaO" verwiesen.

Gefäßimplantate zur Verhinderung und/oder Beseitigung von Gefäßverengungen, die mindestens eine Radionuklid-Spezies enthalten, sind aus nachfolgenden Veröffentlichungen bekannt geworden:
DE-C-4315002
EP-A-0433011
EP-A-0539165

Zur Aktivierung der Gefäßimplantate gemäß der DE 4315002 wird ein externer Strahl eines Zyklotrons mit 9,2 MeV Deuteronen eingesetzt. Durch den Beschuss mit einem gerichteten Deuteronenstrahl wird im CrNi-Stahl des Stents gemäß der DE 4315002 ein hoher Anteil von Radionukliden mit Elektroneneinfang-Übergängen und daraus resultierender weicher Röntgenstrahlung sowie β₊-Übergängen induziert.

Nachteilig an diesem Verfahren ist neben dem hohen Aufwand insbesondere, daß durch den Beschuß mit radioaktiven Strahlteilchen die Aktivierung in einem Volumen bis einige Mikrometer Tiefe erzeugt wird. Damit befinden sich ebenfalls radioaktive Kerne direkt an der Oberfläche. Diese können beim Kontakt mit beispielsweise Blut austreten und zu einer radioaktiven Gefährdung des Patienten führen.

Ein weiterer Nachteil ist die geringe Ausbeute bei der Bestrahlung.

Daher erfordert die Herstellung eines Gefäßimplantates gemäß dem Stand der Technik in Form der DE 4315002 eine sorgfältige und intensive Nachbehandlung der Oberflächen, um ein unkontrolliertes Entweichen von Radioaktivität in den Körper zu verhindern.

Aufgabe der Erfindung ist es somit, ein gegenüber dem Stand der Technik verbessertes Schichtsystem anzugeben, dass sich durch eine hohe Biokompatibilität auszeichnet und bei Bestrahlung beispielsweise mit Neutronen zur Erzeugung eines radioaktiven Gefäßimplantates die Nachteile des Standes der Technik vermeidet.

Erfindungsgemäß wird diese Aufgabe dadurch gelöst, dass bei einem System mit einem Substrat als Träger und einer auf das Substrat aufgebrachten Beschichtung die Beschichtung wenigstens die Elemente Titan (Ti) oder Aluminium (Al) und Phosphor (P) umfasst.

Als Substratmaterial sind insbesondere alle in der Medizin verwendbaren Materialien, die mit einer Beschichtung versehen werden können, denkbar. Nur beispielhalber seien hier Metalle, Metallegierungen, Gläser und Kunststoffe genannt.

Bevorzugt umfasst die Beschichtung des weiteren Stickstoff (N) sowie ein Metall, das inbesondere aus einem der nachfolgenden Elemente:
Si, Ge, W, Pt, Pb, Co, Sm, Ir, Re
ausgewählt wird.

Der Phosphoranteil der Beschichtung beträgt bevorzugt maximal 35 Atom-%, wobei eine Zusammensetzung mit weniger als 5 Atom-% Phosphor bereits zu einer guten Haftung auf dem Substrat und einer Stresserniedrigung der Beschichtung führt. Bei einem Phosphorgehalt zwischen 5 und 10 Atom-% werden zusätzlich zur Stresserniedrigung in der Schicht Verschleißeigenschaften ähnlich dem TiN erreicht.

Soll die Beschichtung eine hohe mechanische Belastbarkeit aufweisen, so wird bevorzugt ein Gewichtsanteil von 10 Atom-% Phosphor (P) eingestellt. Schichten mit einem Anteil von 20 Atom-% bis 35 Atom-% Phosphor weisen eine niedrigere mechanische Belastbarkeit auf. Der hohe Phosphorgehalt, wie nachfolgend beschrieben, kann mit Hilfe eines geeigneten Neutronenflusses aktiviert werden.

Die Schichtdicken der erfindungsgemäßen Beschichtung liegen zwischen 10 Nanometer (nm) und 20 Mikrometer (µm), wobei Schichtdicken bis 0,5 Mikrometer zur Erzeugung der Biokompatibilität dienen und Schichtdicken zwischen 0,5 Mikrometer und 20 Mikrometer für die Neutronenaktivierung bei der Erzeugung eines radioaktiven Stents eingesetzt werden können.

Um die Schichten als Träger von Medikamenten einsetzen zu können, ist es vorteilhaft, wenn die Schichten eine hohe Porosität, bevorzugt zwischen 1 bis 4 Mikrometer aufweisen. Die Porosität der Schicht kann durch die Wahl der Beschichtungsparameter Substratwinkel, Substrattemperatur, Beschleunigungsspannung und Plasmaleistung eingestellt werden.

Ein Beschichtungsverfahren wird bevorzugt mit Hilfe einer Magnetron-Sputteranlage mit einem Prozessgas aus Ar-N hergestellt.

Als Träger für die Beschichtung werden ganz bevorzugt Materialien für Gefäßimplantate aus reinem Titan oder Gedächtnislegierungen beispielsweise Nitinol oder eine Ni-Ti-Legierung verwandt. Prinzipiell können als Trägermaterialien bei der Verwendung derartiger Systeme zur Erzeugung radioaktiver Implantate alle derartigen Elemente zum Einsatz kommen, die beim Beschuss mit thermischen Neutronen keine bzw. nur eine sehr geringe Anzahl aktiver Isotope bilden und deren Aktivitätsprodukte bereits nach einer kurzen Abklingzeit, die bevorzugt im Bereich weniger Stunden liegt, auf ein zu vernachlässigendes Maß abgeklungen sind.

In einer besonders vorteilhaften Ausgestaltung der Erfindung wird auf den Träger zunächst gleichmäßig eine Schicht aus Ti-Al-N-P, Ti-N-P bzw. Al-N-P aufgebracht, und zwar mit einer Schichtdicke im Bereich zwischen 1 und 3 Mikrometer.

Auf diese Schicht wird in einem zweiten Beschichtungsprozess eine dünne Deckschicht aus TiN oder amorphen Kohlenstoff aufgebracht, um die darunterliegende Funktionsschicht aus Ti-Al-N-P, Ti-N-P bzw. Al-N-P abzuschließen. Die Schichtdicke der zweiten Schicht beträgt nur wenige Nanometer und zeichnet sich durch eine besonders gute Biokompatibilität aus. Die zweite Schicht bildet eine Diffusionssperre für die darunterliegende aktivierte Funktionsschicht.

Neben dem Beschichtungssystem gibt die Erfindung auch ein Verfahren zur Beschichtung eines Substrates mit einer Beschichtung umfassend Titan und Phosphor durch Aufsputtern in einer Sputteranlage, umfassend eine Vakuumkammer, an.

Ein oberbegriffliches Verfahren zum Aufsputtern in einer Sputteranlage ist aus dem Stand der Technik beispielsweise in Form von Dieter Sautter, Hans Weimerth, "Lexikon Elektronik und Mikroelektronik", S. 804 - 806, aaO, hinlänglich bekanntgeworden.

Das erfindungsgemäße Verfahren zeichnet sich dadurch aus, dass als Sputtertarget ein Ti-Ti₂-P-Mₓ- oder Ti-Ti₂-P-Mischtarget verwendet wird, wobei Mₓ ein Metall, bevorzugt Si, Ge, W, Pt, Pb, Co, Sm, Ir, Re ist, bzw. ein Al-Ax-P-Mischtarget, wobei Ax ein oder mehrere beliebige Elemente bedeutet.

In einer bevorzugten Ausführungsform der Erfindung handelt es sich bei dem erfindungsgemäßen Sputtertarget um ein Target mit einem Titan-Grundkörper, in den Bohrungen eingelassen sind. In diese Bohrungen wiederum werden Pellets eingebracht, die beispielsweise aus einem Ti-P-Al-Pulvergemisch unter hohem Druck gepresst werden. Die Stöchiometrie des Targets, beispielsweise die Anzahl der Pellets, wird nunmehr so eingestellt, dass bei einer niedrigen Prozesstemperatur von beispielsweise 200° C eine sehr harte Schicht von beispielsweise 1800 Vickers Härte mit einem kleinen Stressverhalten - kompressiver Stress - von beispielsweise 1,5 GPa beim Aufsputtem entsteht. Bevorzugt liegt der P-Gehalt in der Schicht bei 4 bis 12 Atom-%.

Als Prozessgas wird bevorzugt Argon bei einem Druck von 1 - 5 x 10⁻³ mbar eingesetzt. Bevorzugt wird das Verfahren so geführt, dass als weiteres Prozessgas Stickstoff mit einem Druck von 1 - 5 x 10⁻³ mbar in die Vakuumkammer eingeleitet wird.

Während der Beschichtung wird das Substrat bevorzugt auf einer Spannung von 15 bis 25 Volt gehalten.

Das erfindungsgemäße Beschichtungssystem findet bevorzugt als Implantat, insbesondere als Gefäßimplantat, beispielsweise als Stent Verwendung, wobei als Beschichtungsverfahren bevorzugt das erfindungsgemäße Verfahren eingesetzt wird.

Die Aktivierung der erfindungsgemäß beschichteten Implantate wird durch Bestrahlung mit Neutronen erreicht. Hierzu wird ein Gefäßimplantat, beispielsweise ein Stent, der aus Nitinol, Titan oder Platin besteht und mit einer 0,5 Mikrometer bis 5 Mikrometer dicken erfindungsgemäßen Beschichtung versehen ist, einem thermischen Neutronenfluss von 10 x 10¹³ bis 5 x 10¹⁵ Neutronen*sec⁻¹*cm⁻² für eine Stunde bis 72 Stunden ausgesetzt. Das in der Beschichtung enthaltene Phosphor ³¹P wird durch den Neutronenbeschuss in das β-aktive Isotop ³²P umgewandelt. Dies entspricht einer β-Aktivität von etwa 50 bis 350 kBq je Implantat, wobei die γ-Aktivität unter 10 kBq liegt.

Die Erfindung soll nachfolgend anhand der Zeichnungen und Ausführungsbeispiele beispielhaft beschrieben werden. Es zeigen
- Fig. 1: ein Nitinol-Stent;
- Fig. 2: ein Querschnitt und ein Längsschnitt eines Stent-Steges aus Fig. 1;
- Fig. 3: Magnetron-Sputteranlage zur Beschichtung eines Substrates mit dem erfindungsgemäßen Verfahren.

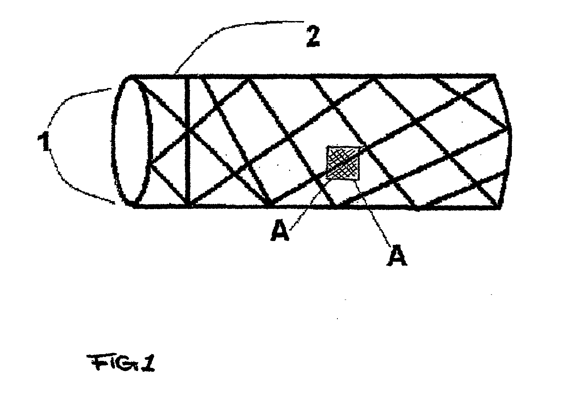

In Fig. 1 ist ein Nitinol-Stent dargestellt. Das Stent umfaßt eine Anzahl von Stegen 2, die netzwerkartig miteinander verbunden sind. Dabei stellt der durch das Netzwerk gebildete Grundkörper 1 einen Zylinder mit kreisrundem Querschnitt dar. Auf die Stege 2 als Substrat ist erfindungsgemäß eine Beschichtung mit den Elementen Titan und Phosphor aufgebracht. Die Stegbreite beträgt 200 Mikrometer.

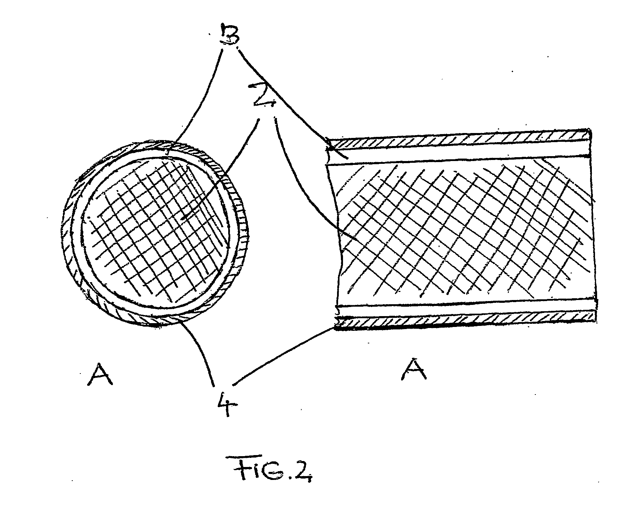

Eine besonders bevorzugte Beschichtung, umfassend eine Funktionsschicht und eine Abschlußschicht, wobei die Abschlußschicht als Diffusionssperre wirkt, wird im Längs- beziehungsweise Axialschnitt durch einen Steg 2 in Figur 2 dargestellt. Man erkennt den Nitinol-Steggrundkörper 2 mit einem Durchmesser von 200 Mikrometer. Direkt auf dem Steggrundkörper 2 ist die phosphorhaltige Titan-Aluminium-Nitridbeziehungsweise Titan-Nitrid-Schicht 3 als Funktionsschicht aufgebracht. Vorzugsweise beträgt die Schichtdicke beim Auftrag etwa 3 Mikrometer und der durch Bestrahlung mit Neutronen aktivierte Phosphoranteil 7 bis 9 Atom-%. Radial um diese Schicht 3 herum erkennt man die nach einer Neutronenaktivierung aktivitätsfreie Titan-Nitrid-Schicht 4, die beim Auftrag eine Dicke von 20 bis 40 Nanometer aufweist und als Diffusionssperre wirkt.

Durch diese Darstellung wird deutlich, daß die β-Strahlung radial und gleichmäßig in das den Stent umgebende Material abgegeben wird.

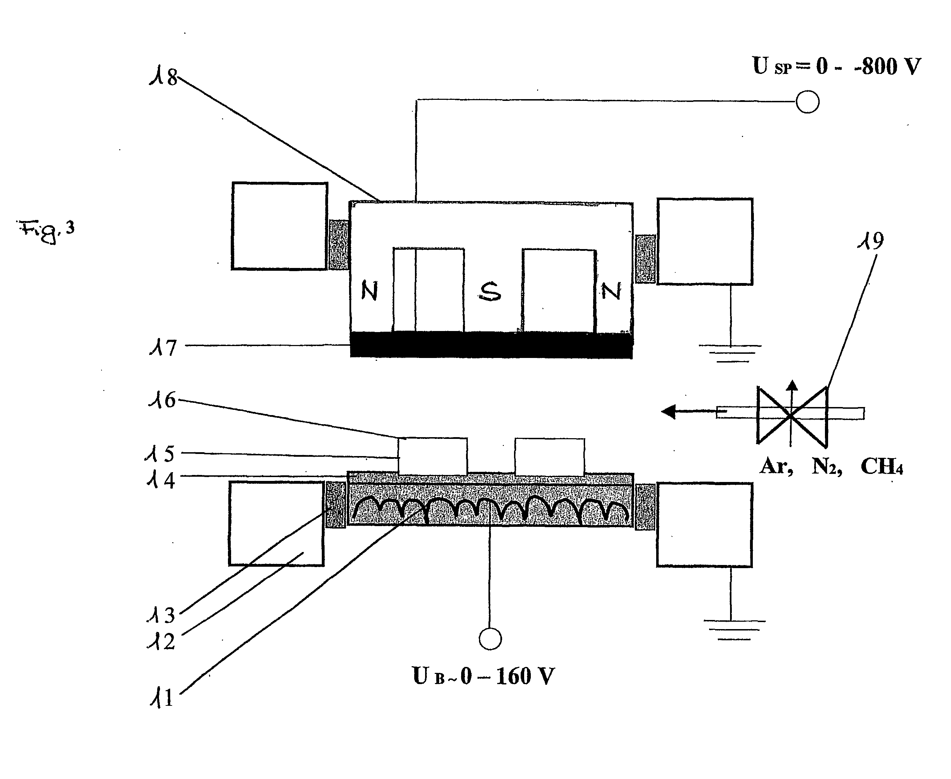

Figur 3 zeigt eine Magnetron-Sputteranlage zur Beschichtung eines Substrates mit dem erfindungsgemäßen Verfahren. Man erkennt einen Rezipienten mit einer rezipienten Wand 12, einem Isolator 13, einem Substratträger 14 und einem Target 17. Der Rezipient kann auf 1 x 10⁻⁶ mbar evakuiert werden. Über ein Ventil 19 kann Prozeßgas in den Rezipienten eingeleitet werden. In dem Rezipienten brennt ein Plasma, in welchem positiv geladene Ionen auf das Target 17 - die Kathode - beschleunigt werden, wobei sich das Target auf einem negativen Potential befindet.

Vom Target 17 werden aufgrund des lonenbeschusses Atome abgelöst, welche sich auf einem Substrat 15 - z. B. einem Stent - ablagern. Dadurch wird der Schichtaufbau erreicht.

Hinter dem Target 17 ist ein Magnet 18 angeordnet, der das Plasma vor der Kathode konzentriert. Durch die damit zu erreichende höhere lonendichte kann eine höhere Abtragungsrate erreicht werden, welche wiederum zu einem schnelleren Schichtwachstum führt.

Vorteilhaft kann am Substrat eine Heizung 11 angeordnet sein, durch die das Schichtwachstum beeinflußt werden kann.

Nachfolgend wird zur weiteren Erklärung der Erfindung die Ausführung eines erfindungsgemäß beschichteten Stents beschrieben:

### Ausführungsbeispiel 1:

Es wurden kommerziell erhältliche Stents der Firma MeKo aus einer Nickel-Titan-Legierung - Nitinol - beschichtet. Die Stents hatten eine Länge von 25 mm, einen Durchmesser von 5 mm im expandierten Zustand, wobei es sich um einen selbstexpandierenden Stent handelte. Die Materialdicke der Stege betrug 200 Mikrometer.

Die Beschichtung bestand aus Titan-Aluminium-Phosphor-Nitrid bzw. aus Titan-Phosphor-Nitrid, wobei die Beschichtung wie folgt erfolgte:

Zunächst wurde der Rezipient auf einen Restdruck von 1 x 10⁻⁵ Millibar evakuiert. Anschließend wurde Argon mit einem Druck von p(Ar) = 1 x 10⁻² Millibar eingelassen, um das Substrat zu reinigen - Sputterreinigung -.

Im eigentlichen Beschichtungsschritt wurde das Argon auf p(Ar) = 1,8 x 10⁻³ Millibar reduziert und gleichzeitig Stickstoff mit einem Druck von p(N) = 8 x 10⁻⁴ Millibar eingelassen.

Das Target war ein TiP-Mischtarget, wobei der Phosphoranteil 15 % betrug, die Sputterspannung bei plus 400 Volt lag und das Substrat an eine Biasspannung von - 15 Volt gelegt wurde.

Bei diesen Bedingungen ergab sich eine Aufdampfrate von 1 Mikrometer/Stunde. Die Substrattemperatur lag bei 240° C.

Der Phosphor, wobei es sich um das Isotop mit der Massenzahl M = 31 handelte, hatte einen Anteil von 7 bis 9 Atom-% an der Beschichtung. Als äußere Schicht wurde eine etwa 20 bis 40 Nanometer dicke reine Titan-Nitrid-Schicht aufgebracht.

Die beschichteten Stents wurden einem thermischen Neutronenfluß von 3x10¹³/Sekunde 24 Stunden lang ausgesetzt. Der Fluß schneller Neutronen lag um einen Faktor 500 niedriger.

Durch Neutroneneinfang wird das Phosphorisotop ³¹P mit einem Neutroneneinfangquerschnitt von 0,16 barn in das β-aktive Isotop ³²P mit einer Halbwertszeit von 14,26 Tagen umgewandelt.

Durch die direkte n,p-Kernreaktion mit schnellen Neutronen werden im wesentlichen durch die Verunreinigungen im Titan und durch die Nickelisotope weitere, kurzlebige γ-aktive Radioisotope erzeugt. Die Hauptaktivität liegt im Röntgenbereich und ist nach einer Abklingzeit von 3 Tagen auf ein der natürlichen Aktivität vergleichbares Maß gesunken. Dabei wurde die Aktivität wie folgt gemessen:

Die aktivierten Proben wurden nach einem, sieben und dreißig Tagen nach erfolgter Aktivierung am Reaktor mit einem hochauflösenden Röntgen- und einem Gamma-Detektor (Germanium) vermessen. Der Abstand zwischen Probe und Detektor betrug 5 cm. Die Meßwerte wurden unter Berücksichtigung des Detektorwirkungsgrades und des Raumwinkels korrigiert, so daß die angegebenen Werte als Gesamtaktivitäten zu verstehen sind.

Die β-Anfangsaktivität betrug 50 kBq. Die Restaktivität aller anderen radioaktiven Isotope betrug unter 1 kBq.

Durch die Neutronenaktivierung der am Substrat angrenzenden Schicht wird erreicht, daß der Stent absolut gleichmäßig aktiviert wird. Die außenliegende Ti-Ni-Schicht bzw. DLC (aC:H) wirkt als Diffusionssperre und verhindert das Austreten radioaktiver Isotope in den Organismus.

Mit der Erfindung wird erstmals ein Schichtsystem angegeben, das auf einfache Art und Weise radioaktiv aktiviert werden kann, sich durch eine hohe Biokompatibilität, geringe Bakterienanhaftung, hervorragende mechanische Eigenschaften sowie geringen Verschleiß auszeichnet. Des weiteren kann das Gefäßimplantat bei Nachlassen der Radioaktivität in einem Reaktor erneut bestrahlt werden und ist so wiederverwendbar.

## Patentansprüche

1. Medizinischer Artikel mit
einem Substrat als Träger
einer auf das Substrat aufgebrachten Beschichtung,
**dadurch gekennzeichnet, dass**
die Beschichtung wenigstens die Elemente Titan (Ti) bzw. Aluminium (Al) und Phosphor (P) umfasst und die Beschichtung eine neutronenaktivierbare phosphorhaltige Funktionsschicht sowie ein über der Funktionsschicht angeordnete Deckschicht aufweist, wobei die Deckschicht nach einer Neutronenaktivierung aktivitätsfrei ist.

2. Medizinischer Artikel nach Anspruch 1, **dadurch gekennzeichnet, dass** die neutronenaktivierbare phosphorhaltige Funktionsschicht eine Ti-Al-N-P, eine Ti-N-P oder eine Al-N-P-Schicht ist.

3. Medizinischer Artikel nach Anspruch 1 oder 2, **dadurch gekennzeichnet**, die Deckschicht eine TiN oder eine amorphe Kohlenstoffschicht ist.

4. Medizinischer Artikel nach einem der Ansprüche 1 bis 3, **dadurch gekennzeichnet, daß** das Substratmaterial ein Material für Gefäßimplantate, insbesondere reines Titan, eine Gedächtnislegierung wie Nitinol oder eine Ni-Ti-Legierung ist.

5. Medizinischer Artikel nach einem der Ansprüche 1 bis 4, **dadurch gekennzeichnet, dass** die Beschichtung eines oder mehrere der nachfolgenden Elemente umfasst:
Si, Ge, W, Pt, Pb, Co, Sm, Ir, Re.

6. Medizinischer Artikel nach einem der Ansprüche 1 bis 5, **dadurch gekennzeichnet, dass** der Phosphoranteil der Funktionsschicht maximal 35 Atom-% beträgt.

7. Medizinischer Artikel nach Anspruch 6, **dadurch gekennzeichnet, dass** der Phosphoranteil der Funktionsschicht maximal 5 Atom-%, bevorzugt maximal 10 Atom-%, ganz bevorzugt maximal 20 Atom-%, insbesondere bevorzugt maximal 35 Atom-% beträgt.

8. Medizinischer Artikel nach einem der Ansprüche 1 bis 7, **dadurch gekennzeichnet, dass** die Schichtdicke der Beschichtung im Bereich 10 Nanometer (nm) - 20 Mikrometer (µm) liegt.

9. Medizinischer Artikel nach einem der Ansprüche 1 bis 8, **dadurch gekennzeichnet, dass** die Beschichtung eine Porösität im Bereich 1 bis 4 Mikrometer aufweist.

10. System nach einem der Ansprüche 1 bis 9, **dadurch gekennzeichnet, dass** die Beschichtung eine Sputter-Reschichtung ist.

11. Verfahren zur Beschichtung eines Substrates eines medizinischen Artikels mit einer Beschichtung umfassend Titan (Ti) bzw. Aluminium (Al) und Phosphor (P), wobei die Beschichtung eine neutronenaktivierbare phosphathaltige Funktionsschicht sowie eine über der Funktionsschicht angeordnete Deckschicht aufweist und die Deckschicht nach einer Neutronenaktivierung aktivitätsfrei ist, durch Aufsputtem in einer Sputteranlage, umfassend eine Vakuumkammer mit folgenden Schritten:
die Vakuumkammer wird evakuiert
es wird wenigstens ein Prozessgas eingeleitet
das Plasma in der Vakuumkammer wird gezündet
die Gasionen des Plasmas sputtem vom in der Vakuumkammer befindlichen geeignet positionierten Sputtertarget die Substanzen, mit denen das Substrat beschichtet wird, heraus, wobei
als Sputtertarget ein Ti-Ti₂-P-Mₓ-Mischtarget und/oder Ti-Ti₂-P-Mischtarget und/oder Al-P-Mischtarget verwendet wird, wobei Mₓ ein Metall, bevorzugt eines der nachfolgenden Metalle
Si, Ge, W, Pt, Pb, Co, Sm, Ir, Re.
ist.

12. Verfahren nach Anspruch 11 **dadurch gekennzeichnet dass** als Prozessgas Ar mit einem Druck von 1 - 7 x 10⁻³ mbar eingeleitet wird.

13. Verfahren nach einem der Ansprüche 11 bis 12, **dadurch gekennzeichnet, dass** als weiteres Prozessgas N₂ mit einem Druck von 0,5 - 6 x 10⁻³ mbar eingeleitet wird.

14. Verfahren nach einem der Ansprüche 11 bis 13, **dadurch gekennzeichnet, dass** das Substrat auf eine Spannung von 15 - 25 V gelegt wird.

15. Implantat, insbesondere Gefäßimplantat, **dadurch gekennzeichnet, dass** das Implantat einen medizinischen Artikel gemäß einem der Ansprüche 1 bis 10 umfasst.

16. Implantat, insbesondere Gefäßimplantat, **dadurch gekennzeichnet, dass** das Implantat eine Beschichtung. hergestellt nach einem Verfahren gemäß einem der Ansprüche 11 bis 14 umfasst.

17. Radioaktives Implantat, wobei das radioaktive Implantat durch Bestrahlen eines Implantates gemäß einem der Ansprüche 15 bis 16 mit einem thermischen Neutronenfluss von 1 x 10¹³ - 5 x 10¹⁵ Neutronen erfolgt.

18. Radioaktives Implantat nach Anspruch 17, **dadurch gekennzeichnet, daß** das Verhältnis des Anteils thermischer Neutronen zum Anteil schneller Neutronen > 100 ist.

19. Radioaktives Implantat nach einem der Ansprüche 17 bis 18, **dadurch gekennzeichnet, dass** die Bestrahlungsdauer zwischen einer Stunde und 72 Stunden liegt.

20. Radioaktives Implantat nach einem der Ansprüche 17 bis 19, **dadurch gekennzeichnet, dass** die Bestrahlung derart erfolgt, dass die Dosisleistung zwischen 0 Gy und 250 Gy/cm² Implantatoberfläche liegt.

21. Radioaktives Implantat nach einem der Ansprüche 17 bis 20, **dadurch gekennzeichnet, dass** das radioaktive Implantat eine β-aktives Implantat ist.

## Claims

1. A medical article comprising
a substrate as carrier,
a coating deposited on the substrate,
**characterized in that**
the coating comprises at least the elements titanium (Ti) or aluminum (Al), respectively, and phosphorus (P) and that the coating has a neutron activateable, phosphorus containing, functional coating, as well as an overcoat arranged above the functional coating, with the overcoat being free of activity after neutron activation.

2. The medical article according to Claim 1, **characterized in that** the neutron activateable, phosphorus containing, functional coating is a Ti-Al-N-P, a Ti-N-P or an Al-N-P coating.

3. A medical article according to Claim 1 or 2, **characterized in that** the overcoat is a TiN or an amorphous carbon coating.

4. A medical article according to one of Claims 1 to 3, **characterized in that** the substrate material is a material for vascular implants, in particular pure titanium, a memory alloy such as nininol or a Ni-Ti alloy.

5. A medical article according to one of Claims 1 to 4, **characterized in that** the coating comprises one or more of the following elements:
Si, Ge, W, Pt, Pb, Co, Sm, Ir, Re.

6. A medical article according to one of Claims 1 to 5, **characterized in that** the proportion of phosphorus of the functional coating amounts to a maximum of 35 atomic %.

7. The medical article according to Claim 6, **characterized in that** the proportion of phosphorus of the functional coating amounts to a maximum of 5 atomic %, preferably a maximum of 10 atomic %, quite preferably a maximum of 20 atomic %, in particular preferably a maximum of 35 atomic %.

8. A medical article according to one of Claims 1 to 7, **characterized in that** the thickness of the coating lies in the range of from 10 nanometers (nm) to 20 micrometers (µm).

9. A medical article according to one of Claims 1 to 8, **characterized in that** the coating exhibits a porosity in the range of from 1 to 4 micrometers.

10. A system according to one of Claims 1 to 9, **characterized in that** the coating is a sputter coating.

11. A process for coating a substrate of a medical article with a coating comprising titanium (Ti) or aluminum (Al), respectively, and phosphorus (P), with the coating having a neutron activateable, phosphorus containing, functional coating as well as an overcoat arranged above the functional coating and the overcoat is free of activity after a neutron activation, by sputter deposition in a sputter plant, comprising a vacuum chamber with the following steps:
the vacuum chamber is evacuated,
at least one process gas is introduced,
the plasma in the vacuum chamber is ignited,
the gas ions of the plasma sputter the substances, with which the substrate is coated, from the sputter target located and suitably positioned in the vacuum chamber, with
a Ti-Ti₂-P-Mₓ mixed target and/or a Ti-Ti₂-P mixed target and/or an Al-P mixed target being used as sputter target, with Mₓ being a metal, preferably one of the following metals:
Si, Ge, W, Pt, Pb, Co, Sm, Ir, Re.

12. The process according to Claim 11, **characterized in that** Ar is introduced as process gas with a pressure of 1 - 7 x 10⁻³ mbar.

13. A process according to one of Claims 11 to 12, **characterized in that** N₂ is introduced as a further process gas with a pressure of 0.5 - 6 x 10⁻³ mbar.

14. A process according to one of Claims 11 to 13, **characterized in that** the substrate is biased with a voltage of 15 to 25 V.

15. An implant, in particular a vascular implant, **characterized in that** the implant comprises a medical article according to one of Claims 1 to 10.

16. An implant, in particular a vascular implant, **characterized in that** the implant comprises a coating produced in accordance with a process according to one of Claims 11 to 14.

17. A radioactive implant with the radioactive implant occurring by irradiation of an implant according to one of claims 15 to 16 with a thermal neutron flux of 1 x 10¹³ - 5 x 10¹⁵ neutrons.

18. The radioactive implant according to Claim 17, **characterized in that** the ratio of the proportion of thermal neutrons to the proportion of fast neutrons is > 100.

19. A radioactive implant according to one of claims 17 to 18, **characterized in that** the duration of irradiation lies between one hour and 72 hours.

20. A radioactive implant according to one of claims 17 to 19, **characterized in that** the irradiation occurs in such a manner that the dose rate lies between 0 Gy and 250 Gy/cm² implant surface.

21. A radioactive implant according to one of Claims 17 to 20, **characterized in that** the radioactive implant is a β-active implant.

## Revendications

1. Article médical avec
un substrat servant de support
pour une enduction apposée sur le substrat,
qualifié par le fait que
l'enduction comporte au minimum comme éléments du titane (Ti) ou de l'aluminium (Al) et du phosphore (P) et que l'enduction présente une couche fonctionnelle à teneur en phosphore activable sous l'action de neutrons ainsi qu'une couche de couverture placée au-dessus de la couche fonctionnelle, à l'occasion de quoi la couche de couverture est exempte d'activité après l'activation aux neutrons.

2. Article médical aux termes de la revendication 1, **caractérisé par le fait que** la couche fonctionnelle à teneur en phosphore activable sous l'action de neutrons est une couche de Ti-Al-N-P, de Ti-N-P ou d'Al-N-P.

3. Article médical aux termes de la revendication 1 ou 2, **caractérisé par le fait que** la couche de couverture est une TiN ou une couche de carbone amorphe.

4. Article médical aux termes des revendications 1 à 2, **caractérisé par le fait que** le matériau du substrat est un matériau destiné aux implants vasculaires, en particulier du titane pur, un alliage de mémoire comme le Nininol ou un alliage Ni-Ti.

5. Article médical aux termes des revendications 1 à 4, **caractérisé par le fait que** l'enduction englobe un ou plusieurs des éléments ci-après :
Si, Ge, W, Pt, Pb, Co, Sm, Ir, Re.

6. Article médical aux termes des revendications 1 à 5, **caractérisé par le fait que** le taux de phosphore de la couche fonctionnelle comporte au maximum 35 % d'atomes.

7. Article médical aux termes de la revendication 6, **caractérisé par le fait que** le taux de phosphore de la couche fonctionnelle comporte au maximum 5 % d'atomes, de préférence au maximum 10 % d'atomes et tout à fait de préférence au maximum 20 % d'atomes, en particulier de préférence au maximum 35 % d'atomes.

8. Article médical aux termes des revendications 1 à 2, **caractérisé par le fait que** l'épaisseur de couche de l'enduction est comprise entre un ordre de grandeur de 10 nanomètres (nm) à 20 micromètres (µm).

9. Article médical aux termes des revendications 1 ou 8, **caractérisé par le fait que** l'enduction présente une porosité d'un ordre de grandeur de 1 à 4 micromètres.

10. Système aux termes de l'une des revendications 1 à 9, **caractérisé par le fait que** l'enduction est une enduction obtenue par pulvérisation (sputter).

11. Procédé d'enduction d'un substrat pour un article médical avec une enduction englobant du titane (Ti) ou de l'aluminium (Al) et du phosphore (P), à l'occasion de quoi l'enduction présente une couche fonctionnelle à teneur en phosphore activable sous l'action de neutrons ainsi qu'une couche de couverture placée au-dessus de la couche fonctionnelle, la couche de couverture étant exempte d'activités après une activation aux neutrons, par pulvérisation (sputter) dans une installation de pulvérisation, englobant une chambre sous vide, avec les étapes suivantes :
la chambre sous vide est évacuée,
au minimum un gaz de processus est injecté,
le plasma dans la chambre sous vide est allumé,
les ions de gaz du plasma pulvérisent, depuis la cible de pulvérisation positionnée de façon appropriée dans la chambre sous vide, les substances avec lesquelles le substrat doit être enduit, à l'occasion de quoi on utilise, comme cible de pulvérisation une cible mixte Ti-Ti₂-P-Mₓ ou une cible mixte Ti-Ti₂-P-Mischtarget ou une cible mixte Al-P, à l'occasion de quoi Mₓ est un métal, de préférence l'un des métaux suivants :
Si, Ge, W, Pt, Pb, Co, Sm, Ir, Re.

12. Procédé aux termes de la revendication 11, **caractérisé par le fait que** le gaz de processus injecté est de l'Ar à une pression de 1 - 7 x 10⁻³ mbars.

13. Procédé aux termes de l'une des revendications 11 à 12, **caractérisé par le fait que** l'on injecte comme autre gaz de processus du N₂ à une pression de 0,5 - 6 x 10⁻³ mbars.

14. Procédé aux termes de l'une des revendications 11 à 13, **caractérisé par le fait que** le substrat est soumis à une tension de 15 - 25 V.

15. Implant, en particulier implant vasculaire, **caractérisé par le fait que** l'implant englobe un article médical aux termes de l'une des revendications 1 à 10.

16. Implant, en particulier implant vasculaire, **caractérisé par le fait que** l'implant englobe une enduction fabriquée selon un procédé aux termes de l'une des revendications 11 à 14.

17. Implant radioactif, à l'occasion de quoi l'implant radioactif est réalisé par exposition aux rayons d'un implant aux termes de l'une des revendications 15 à 16 avec un influx de neutrons thermiques de 1 x 10¹³ - 5 x 10¹⁵ neutrons.

18. Implant radioactif aux termes de la revendication 17, **caractérisé par le fait que** le rapport entre le taux de neutrons thermiques et le taux de neutrons rapides est > 100.

19. Implant radioactif aux termes de l'une des revendications 17 à 18, **caractérisé par le fait que** la durée d'exposition aux rayons est comprise entre une heure et 72 heures.

20. Implant radioactif aux termes de l'une des revendications 17 à 19, **caractérisé par le fait que** l'exposition aux rayons s'effectue de telle manière que la puissance de dosage soit comprise entre 0 Gy et 250 Gy/cm² de surface d'implant.

21. Implant radioactif aux termes de l'une des revendications 17 à 20, **caractérisé par le fait que** l'implant radioactif est un implant β-actif.
